# EUROPEAN PATENT APPLICATION

(11) **EP 4 529 845 A1**
(43) Date of publication of application: **02.04.2025**
(21) Application number: 23811939.0
(22) Date of filing: 14.02.2023
(51) Int. Cl.: A61B 6/00, A61B 6/03, A61B 5/00, G06T 17/00, G06T 7/00

(54) **AUTOMATIC THREE-DIMENSIONAL FACE SCAN MATCHING DEVICE HAVING ARTIFICIAL INTELLIGENCE APPLIED THERETO, METHOD FOR DRIVING SAME, AND COMPUTER PROGRAM STORED IN MEDIUM**

(30) Priority: 23.05.2022 KR 20220062951
(71) Applicant: Megagen Implant Co., Ltd., Dalseong-gun, Daegu 42921 (KR)
(72) Inventor: PARK, Kwang Bum, Daegu 42016 (KR); LEE, Tae Gyoun, Daegu 41535 (KR); SO, Jae Hong, Changwon-si Gyeongsangnam-do 51484 (KR)
(74) Representative: Patentanwälte Hemmer Lindfeld Frese Partnerschaft mbB
(86) International application number: PCT/KR2023/002140
(87) International publication number: WO 2023/229152

(57) **Abstract**

Provided are an automatic three-dimensional face scan matching device having artificial intelligence applied thereto, a method for driving the device, and a computer program stored in a medium. According to the present inventive concept, the automatic three-dimensional face scan matching device having artificial intelligence applied thereto includes a communication interface unit receiving first image data obtained by capturing a face center portion during computed tomography (CT) scanning of a face of a user and second image data obtained by scanning the face of a user in a 3D scanning method, and a control unit visualizing the face of a user into 3D by analyzing the first image data and the second image data by having artificial intelligence (AI) applied thereto to find detection points of landmarks that are commonly detected, converting two-dimensional coordinate values for the detection points found on the second image data into three-dimensional coordinate values, and automatically matching the second image data to CT scanning image data based on the converted three-dimensional coordinate values.

## Description

### TECHNICAL FIELD

The present inventive concept relates to an automatic three-dimensional face scan matching device having artificial intelligence applied thereto and a method for driving the device, and more particularly, to an automatic three-dimensional face scan matching device having artificial intelligence applied thereto, which enables seeing computed tomography (CT) for a smiling face of a user by visualizing the CT image into three-dimensions (3D), for example, during a dental treatment or a cosmetic treatment, so as to increase the accuracy of diagnosis, a method for driving the device, and a computer program stored in a medium.

### BACKGROUND ART

An implant originally means a substitute used to restore lost human tissues. In a dental field; however, the implant refers to a series of surgeries to implant an artificial tooth. The implant is a series of surgeries to restore the function of a tooth by placing a fixture on an alveolar bone where a tooth is lost, and then fixing an artificial tooth thereon. The fixture is a tooth root made of titanium etc. having no rejection by the human body to replace a lost tooth root (root). For general prostheses or dentures, the surrounding teeth and bone are damaged as time passes, whereas the implant does not damage the surrounding teeth tissues, has the same function and shape as a natural tooth, and does not cause cavities, and thus, it is advantageous that the implant can be used semi-permanently.

In the artificial tooth surgery (or referred to as an implant or an implant surgery), it is general to make, by using a drill, a screw hole in an alveolar bone at a placement position where a fixture is to be placed, place the fixture into the screw hole to be osseointegrated with the bone to form an artificial tooth root, couple an abutment to the fixture, and then completely cap the abutment with a final prosthesis. The dental implant not only restores a single missing tooth, but also improves the function of dentures for partially edentulous and completely edentulous patients, and improves the aesthetic aspect of dental prosthetic restorations. Furthermore, the implant distributes excessive stress applied to the surrounding supporting bone tissues and also helps the stabilization of the dentition.

Meanwhile, in order to increase the accuracy of an implant procedure in an implant procedure process, a simulation procedure and procedure planning are involved. Accurate data for an oral cavity area of one subject to a procedure is essential for the simulation procedure and the procedure planning. Generally, to obtain the data for oral cavity area of one subject to a procedure, a method of obtaining three-dimensional image data by capturing an image of the oral cavity area of one subject to a procedure using a computed tomography (CT) apparatus is used.

While the computed tomography has an advantage of being able to see the actual proportions of a user, that is, the patient's face, in 3D, due to radiation exposure, it is generally recommended to close the eyes and the mouth when taking scanning. For the actual dental or cosmetic treatment, however, it is important to reflect the smiling face of a user, and thus, a method for matching the position of a user (e.g.: teeth in an oral cavity, etc.) on a smiling face is urgently required.

### DESCRIPTION OF EMBODIMENTS

### TECHNICAL PROBLEM

It is an objective of the present inventive concept to provide an automatic three-dimensional face scan matching device having artificial intelligence applied thereto, which enables seeing computed tomography for a smiling face of a user by visualizing a CT image into 3D, for example, during a dental treatment or a cosmetic treatment, so as to increase the accuracy of diagnosis, a method for driving the device, and a program stored in a medium.

### ADVANTAGEOUS EFFECTS OF DISCLOSURE

According to an embodiment of the present inventive concept, during a dental treatment or a cosmetic treatment, a CT image can be seen by being visualized in 3D by reflecting a smiling face of a user, and thus, accuracy of diagnosis may be increased.

Furthermore, according to an embodiment of the present inventive concept, when image data by CT scanning and scan data of scanning a smiling face of a user, which are data with heterogeneity, are combined with each other, an artificial intelligence program is applied thereto, and thus, accuracy and rapidity of data processing may be increased.

### BRIEF DESCRIPTION OF DRAWINGS

FIG. 1 is a block diagram showing an example of a detailed structure of an Al-based automatic 3D face scan matching device according to an embodiment of the present inventive concept.
FIG. 2 is a view for explaining data configurations of a SCOUT image and a face scan image.
FIG. 3 is a block diagram showing an example of a detailed structure of Al-based automatic 3D face scan matching unit of FIG. 1.
FIG. 4 is a view for explaining a comparison between a manual method and an automatic method having artificial intelligence applied thereto when performing marking (or detection) on a detection point.
FIG. 5 is a view for explaining a detailed operation of a face and landmark detection unit of FIG. 3.
FIG. 6 is a view for explaining operations S500 to S520 of FIG. 5.
FIG. 7 is a view for explaining operation S540 of FIG. 5.
FIG. 8 is a view for explaining a detailed operation of a 3D point detection unit of FIG. 3.
FIG. 9 is a view for explaining operation S800 to operation S850 of FIG. 8.
FIG. 10 is a flowchart showing a driving process of the Al-based automatic 3D face scan matching device of FIG. 1.

### BEST MODE

According to an aspect of the present inventive concept, there is provided an automatic three-dimensional face scan matching device having artificial intelligence applied thereto, the device including a communication interface unit receiving first image data obtained by capturing a face center portion during computed tomography (CT) scanning of a face of a user and second image data obtained by scanning the face of a user in a 3D scanning method, and a control unit visualizing the face of a user into 3D by analyzing the first image data and the second image data by having artificial intelligence (AI) applied thereto to find detection points of landmarks that are commonly detected, converting two-dimensional coordinate values for the detection points found on the second image data into three-dimensional coordinate values, and automatically matching the second image data to CT scanning image data based on the converted three-dimensional coordinate values.

The control unit may analyze the first image data and the second image data by having the artificial intelligence applied thereto to extract a plurality of detection points related to feature points respectively therefrom, and extract a plurality of landmark detection points that are common with each other from among the extracted plurality of detection points.

The control unit may extract, as the landmark detection points, detection points where the feature points are not changed in a face portion during the CT scanning and a face portion during the 3D scanning.

The control unit may preset reference information to detect the feature points and the plurality of landmark detection points, and detect the feature points and the landmark detection points by applying the artificial intelligence thereto based on the preset reference information.

The control unit may determine a shape formed with the feature points as the reference information, and finalize the landmark detection points based on a result of the determination.

The control unit may convert the second image data into three-dimensional UV map data, convert two-dimensional coordinate values for the detection points into the three-dimensional coordinate values by using the converted UV map data and three-dimensional data generated during the face scanning, by determining a shape formed with three coordinates during the conversion of the three-dimensional coordinate values and color information of each coordinate, and perform conversion of the three-dimensional coordinate value based on a result of the determination.

Furthermore, according to another aspect of the present inventive concept, there is provided a method for driving a three-dimensional face scan automatic matching device having artificial intelligence applied thereto, the method including receiving, performed by a communication interface unit, first image data obtained by capturing a face center portion during computed tomography scanning of a face of a user and second image data obtained by scanning the face of a user in a 3D scanning method, and visualizing, performed by a control unit, the face of a user into 3D by analyzing the first image data and the second image data by having artificial intelligence applied thereto to find detection points of landmarks that are commonly detected, converting two-dimensional coordinate values for the detection points found on the second image data into three-dimensional coordinate values, and automatically matching the second image data to CT scanning image data based on the converted three-dimensional coordinate values.

The visualizing may include extracting a plurality of detection points related to feature points respectively therefrom by analyzing the first image data and the second image data by having the artificial intelligence applied thereto, and extracting a plurality of landmark detection points that are common with each other from among the extracted plurality of detection points.

The extracting of the plurality of landmark detection points may include extracting, as the landmark detection points, detection points where the feature points are not changed in a face portion during the CT scanning and a face portion during the 3D scanning.

The visualizing may include presetting reference information for detecting the feature points and the plurality of landmark detection points, and detecting the feature points and the landmark detection points by applying the artificial intelligence thereto based on the preset reference information,

The detecting of the landmark detection point may include determining a shape formed with the feature points as the reference information, and finalizing the landmark detection points based on a result of the determination.

The visualizing may include converting the second image data into three-dimensional UV map data, converting two-dimensional coordinate values for the detection points into the three-dimensional coordinate value by using the converted UV map data and three-dimensional data generated during the face scanning, and determining a shape formed with three coordinate values during the conversion of the three-dimensional coordinate values and color information of each coordinate, and performing conversion of the three-dimensional coordinate values based on a result of the determination.

Furthermore, according to another aspect of the present inventive concept, there is provided a computer program stored in a medium to perform a three-dimensional face scan automatic matching method by having artificial intelligence applied thereto, wherein the three-dimensional face scan automatic matching method by having artificial intelligence applied thereto may include receiving first image data obtained by capturing a face center portion during computed tomography scanning of a face of a user and second image data obtained by scanning the face of a user in a 3D scanning method, and visualizing the face of a user into 3D by analyzing the first image data and the second image data by having artificial intelligence applied thereto to find detection points of landmarks that are commonly detected, converting two-dimensional coordinate values for the detection points found on the second image data into three-dimensional coordinate values, and automatically matching the second image data to CT scanning image data based on the converted three-dimensional coordinate values.

### MODE OF DISCLOSURE

Embodiments are provided to further completely explain the disclosure to one of ordinary skill in the art to which the disclosure pertains. However, the disclosure is not limited thereto, and it will be understood that various changes in form and details may be made therein without departing from the spirit and scope of the following claims. That is, descriptions on particular structures or functions may be presented merely for explaining embodiments of the disclosure.

Various modifications may be applied to the present embodiments, and particular embodiments will be illustrated in the drawings and described in the detailed description section. However, various embodiments of the document and terms used therein are not intended to limit the disclosure to particular modes of practice, and it is to be appreciated that various modifications, equivalents, and/or alternatives that do not depart from the spirit and technical scope of the disclosure are encompassed in the disclosure.

Terms such as "first" and "second" are used herein merely to describe a variety of constituent elements, but the constituent elements are not limited by the terms. Such terms are used only for the purpose of distinguishing one constituent element from another constituent element. For example, without departing from the right scope of the disclosure, a first constituent element may be referred to as a second constituent element, and vice versa.

When a constituent element "connects" or is "connected" to another constituent element, the constituent element contacts or is connected to the other constituent element directly or through at least one of other constituent elements. Conversely, when a constituent element is described to "directly connect" or to be "directly connected" to another constituent element, the constituent element should be construed to be directly connected to another constituent element without any other constituent element interposed therebetween. Other expressions, such as, "between" and "directly between," describing the relationship between the constituent elements, may be construed in the same manner.

Terms used in the disclosure are used for explaining a specific embodiment, not for limiting the disclosure. Thus, an expression used in a singular form in the disclosure also includes the in its plural form unless clearly specified otherwise in context. Also, terms such as "include" or "comprise" may be construed to denote a certain characteristic, number, step, operation, constituent element, or a combination thereof, but may not be construed to exclude the existence of or a possibility of addition of one or more other characteristics, numbers, steps, operations, constituent elements, or combinations thereof.

Unless defined otherwise, all terms used herein including technical or scientific terms have the same meanings as those generally understood by those of ordinary skill in the art to which the disclosure may pertain. The terms as those defined in generally used dictionaries are construed to have meanings matching that in the context of related technology and, unless clearly defined otherwise, are not construed to be ideally or excessively formal.

In the drawings, the thicknesses of layers and regions are exaggerated for clarity. It will also be understood that when a layer is referred to as being "on" another layer or substrate, it can be directly on the other layer or substrate, or intervening layers may also be present. Like reference numerals in the drawings denote like elements, and thus their description will not be repeated.

Terms, such as an upper end, a lower end, an upper surface, a lower surface, a front surface, a rear surface, an upper portion, or a lower portion, etc., are used to distinguish a relative position of a component. For example, for convenience, when an upper side of the drawing is referred to as an upper portion and a lower side of the drawing is referred to as a lower portion, actually, without departing from the right scope of the disclosure, an upper portion may be referred to as a lower portion, and a lower portion may be referred to as an upper portion. Furthermore, to help understanding of the disclosure, the accompanying drawings are not illustrated down to actual scale, but are illustrated to be exaggerated in dimensions of some components.

Hereinafter, an embodiment of the present inventive concept is described in detail with reference to the accompanying drawings.

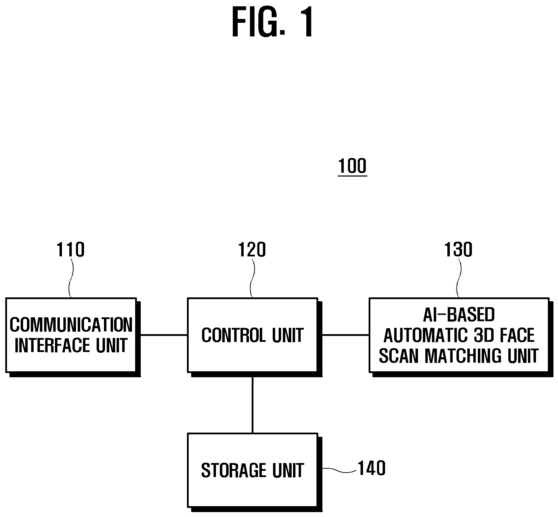

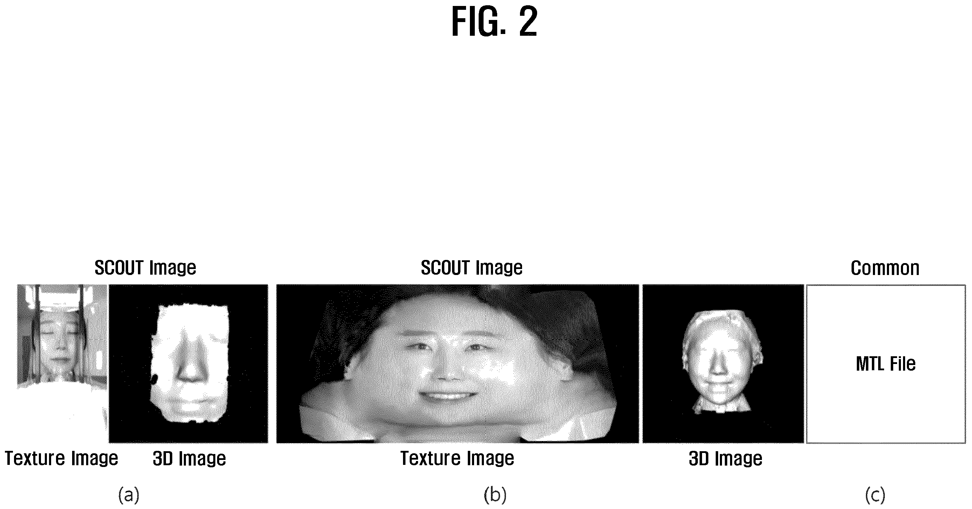

FIG. 1 is a block diagram showing an example of a detailed structure of an Al-based automatic 3D face scan matching device according to an embodiment of the present inventive concept. FIG. 2 is a view for explaining data configurations of a SCOUT image and a face scan image.

As illustrated in FIG. 1, an artificial intelligence (AI) based (or applying) automatic 3D face scan matching device (and software) (hereinafter, referred to as device) 100 according to an embodiment of the present inventive concept includes some or all of a communication interface unit 110, a control unit 120, an Al-based automatic 3D face scan matching unit 130, and a storage unit 140.

Prior to the detailed descriptions, the Al-based automatic 3D face scan matching device 100 according to an embodiment of the present inventive concept may include various apparatuses, such as a desktop computer, a laptop computer, a tablet PC, a smartphone, etc. used in dentistry, etc., but as a CT scanning device for performing CT scanning and a 3D scanning device for 3D scanning the face of a user, that is, a patient, may be included, an embodiment of the present inventive concept may not be particularly limited to any one device. Furthermore, the Al-based automatic 3D face scan matching device 100 may include a computer readable recording medium included in a corresponding device or a computer program (or software) stored in the medium. For example, the 3D scanning device may obtain three-dimensional scan data without distortion as scanning is simultaneously performed from cameras located in three directions. This is illustrated in (b) of FIG. 2. As it is a 3D scanning device, it is obvious to obtain, as shown in (b) of FIG. 2, two-dimensional texture image data and three-dimensional data for a black and white three-dimensional image.

Furthermore, a Cone Beam CT (CBCT) may be used as the CT scanning device according to an embodiment of the present inventive concept. The CBCT is mainly used in dentistry. The CBCT is smaller than a medical CT used to scan in general hospitals, enables upright scanning, and takes a time for about 15 seconds to 30 seconds which is shorter than the medical CT. Tomography is performed through a sensor, that is, a detector, which receives X-ray signals. When X-rays are irradiated, signals reflected by various tissues, organs, etc. in the face are scanned through the detector, and the signals are utilized as depth information, thereby generating three-dimensional data. As such, the CBCT used in dentistry is used to visually see the oral cavity or the head. During CT scanning, or through a separate camera, etc., a capture image of a face center portion of a patient is obtained, which may be referred to as a SCOUT image (or an image). Scanning is performing to find the location of a lesion or a region of interest prior to the main inspection of a patient. In this state, the SCOUT image is used to set a reference for determining an inspection range. In other words, the SCOUT image is an image for scanning to designate a scanning portion by checking the presence or absence of a disease, etc. of a patient before direct inspection. For example, when a scanning operator specifies an area to scan while seeing a SCOUT image, images are continuously captured with a certain thickness within the specified range. Thus, when a line is generated on a SCOUT image by identifying the position of a scanned slice image and then the SCOUT image and the slice image are linked with each other, while browsing images such as CT images, etc., the position of an image may be checked, and when a generated line is clicked, a corresponding slice image is output so that a function of fast accessing a desired image may be assigned.

Images such as CT images, etc. are captured by tens of images at least or hundreds of images at most, for one time, depending on the patient's state or as necessary. Generally, doctors diagnose a patient's state while seeing continuously scanned slice images, and thus, it takes too long to find an abnormal region or a necessary portion from tens of images. Accordingly, by generating a line on a SCOUT image so as to directly click the line, a slice image of a corresponding portion is directly output so that an image of a desired portion may be seen within a fast time. It is most important in generating a SCOUT line to accurately match the position of a captured slice image and the position of a line to be generated. Accordingly, a SCOUT line is generated at an accurate position by distinguishing a correlation between the SCOUT image and each slice image.

The Al-based automatic 3D face scan matching device 100 according to an embodiment of the present inventive concept may perform R2 STUDIO Q scanning. In this state, the generated data may include three types of data of the CBCT image, a SCOUT image as in (a) of FIG. 2, and a face scan image as in (b) of FIG. 2. Here, the "R2 STUDIO Q scanning" may refer to scanning performed by the Al-based automatic 3D face scan matching device 100, that is, a device separately manufactured to perform an operation according to an embodiment of the present inventive concept. The SCOUT image or the 3D face image is three-dimensional data including a texture image file (bmp, png, jpg, etc.), a three-dimensional image file (.obj), and a setting file (.mtl), as can be seen in FIG. 2.

A texture (or texture) image file is a filed obtained by scanning a patient and is a file having color, that is, color information, which may be skin data that is an outer surface of the face. The texture image file is an image used for an OBJ file and used in association with a three-dimensional image. Furthermore, the three-dimensional image file means data formed of white, that is, black and white. The three-dimensional image file is used in an OBJ file format and includes coordinates (value) for expressing in three-dimensions and data by which hue or color may be assigned to a face surface. The three-dimensional image may include V and VT factors. V records coordinate values of three-dimensional coordinates (X, Y, Z), and generates a surface by threes in a group. Accordingly, the three coordinates have a polygon structure. In other words, a polygon structure is formed by three coordinates. VT records texture image coordinates for a color to be assigned to a surface of a three-dimensional image, that is, a face skin surface. For example, by obtaining a texture image file color, the color is applied to a surface. An MTL file includes a system path of a texture image file to be used by a three-dimensional image and setting values of effect.

Based on the descriptions presented above, the communication interface unit 110 of FIG. 1 may operate as a data receiving unit. The communication interface unit 110 receives each of SCOUT data of a SCOUT image and scan data of a face scan image through CBCT scanning or 3D face scanning. For example, data of a SCOUT image may be received through a tomography device such as the CBCT, and data of a face scan image in (b) of FIG. 2 may be received through a 3D face scanning device. Two image data is data having, for example, different, that is, heterogeneous characteristics for the patient's face. While the SCOUT data is data of a scanning image captured when a patient has to close the eyes and mouth to avoid radiation during CT scanning, the face scan image data is data on a face image of a patient smiling as in everyday life, as there is no radiation exposure as in CT scanning.

The control unit 120 is in charge of the overall control operation of the communication interface unit 110, the Al-based automatic 3D face scan matching unit 130, and the storage unit 140 of FIG. 1. When SCOUT image data and 3D face scan image data are received through the communication interface unit 110, the control unit 120 temporarily stores the received data in the storage unit 140 and then retrieves and provides the same to the Al-based automatic 3D face scan matching unit 130.

When the integration (or overlapping) of the CBCT image data and the face scan image is achieved through the Al-based automatic 3D face scan matching unit 130, the control unit 120 may display, on a screen, data that is integrated according to the dentist's command by visualizing the data in 3D. The screen may be a computer's monitor, and may be a separate display unit.

The Al-based automatic 3D face scan matching unit 130 performs an automatic matching operation of the SCOUT image data and the face scan image data obtained by capturing the face center portion of a patient, for example, during CT scanning, and by applying an Al program during the process, may increase accuracy in the matching operation. It may be said that, as mentioned above, such an operation is to match the position of a patient through a smiling face of the patient during an actual dental or cosmetic treatment, while during CT scanning, scanning is performed with the eyes or mouth closed to prevent patient's radiation exposure. In other words, although, in a CT scanning image, scanning is performed while a patient with the eyes and mouth closed, the Al-based automatic 3D face scan matching unit 130 substitutes only image data for the face surface with the face scan image data. In this case, however, the patient's bone structure, tooth locations, etc. when smiling are different from those seen during CT scanning, and thus, it may be said that the differences are corrected based on the face scan image data to achieve 3D visualization. In this state, by converting CT image data based on detection points of landmarks that are commonly detected from the two-dimensional SCOUT image data and the face scan image data, accuracy of data processing may be improved.

In more detail, the Al-based automatic 3D face scan matching unit 130 detects n three-dimensional coordinates having a clinical meaning by using two-dimensional, three-dimensional data. The matching process of the SCOUT data and the face scan data is automated by having artificial intelligence applied thereto, that is, an Al program. For example, feature points for two different faces may be detected from the SCOUT image data and the face scan image data. In an embodiment of the present inventive concept, a face is recognized through detection of, for example, 51 feature points. Then, n landmarks for common portions, that is, portions having no change when closing eyes or smiling, are detected among the detected feature points. The detection points of landmarks may be 4 to 6, up to 51. In an embodiment of the present inventive concept, in this process, a reference point (or reference information) is set at a consistent position through artificial intelligence so as to detect feature points and also accurately detect the landmark. Various pieces of information, such as pixel information or pixel boundary information on an image, or a shape by the pixel information, etc., may be used as the reference information. In an embodiment of the present inventive concept, a portion of the tip of the nose or the tip of the nostrils, etc. corresponds to the landmark. Accordingly, when the reference point is set, image analysis is performed through artificial intelligence and certainly a learning operation is performed, thereby enabling accurately marking a portion of the tip of the nose and the tip of the nostrils, etc. More accurately, it may be preferred to recognize the corresponding detection points based on the reference information, rather than marking. In some cases, this may be expressed as extracting a detection point. Accordingly, by matching a coordinate value (or a first coordinate value) of the SCOUT image data to a coordinate value (or a second coordinate value) of the 3D scan image data, which correspond to the same portion, with each other, when seeing 3D visualization data, a CT image can be seen through a smiling face image. For example, as a conversion relation, etc. may be seen through the two coordinate values, even when the scan image data of a smiling face is matched to or overlapped with three-dimensional CT scanning image data, by converting the CT image data based on the conversion relation, 3D visualization data combined with a smiling face may be generated.

For example, it is assumed that the above marking (or detection point detection) operation is performed manually. As the operation is performed manually, not automatically, if things go wrong, a re-performing operation may be necessary. Furthermore, a lot of time is consumed for data processing due to manual performing. In contrast, as in an embodiment of the present inventive concept, when artificial intelligence is applied thereto, 10-point marking, such as the tip of the nostrils, etc., may be completed by one click, and also, marking may be made at the consistent position, or the detection of consistent position may be possible. Accordingly, a CT image for a smiling face can be seen so that the accuracy of diagnosis increases. It is possible to make 3D visualization of the CT image by combining tomography data with depth information therewith. Detailed content related to the Al-based automatic 3D face scan matching unit 130 is discussed later.

The storage unit 140 temporarily stores data processed under the control of the control unit 120. When the SCOUT image (or image) data and the 3D face scan data are received from the communication interface unit 110, the control unit 120 temporarily stores the received data on the storage unit 140 and then retrieves and provide the same to the Al-based automatic 3D face scan matching unit 130, thereby enabling an automatic matching operation having artificial intelligence applied thereto.

In addition to the above content, the communication interface unit 110, the control unit 120, the Al-based automatic 3D face scan matching unit 130, and the storage unit 140 of FIG. 1 may perform various operations, and other detailed contents are continuously discussed later.

Although the communication interface unit 110, the control unit 120, the Al-based automatic 3D face scan matching unit 130, and the storage unit 140 of FIG. 1, according to an embodiment of the present inventive concept, are configured with hardware modules physically separated from each other, each module may store therein software to perform the operation and execute the same. However, as the software is a group of software modules, and it is possible to form each module by hardware, it may not be particularly limited to a configuration of software or hardware. For example, the storage unit 140 may be a storage or a memory that is hardware. However, as it is also possible to store (repository) information software-wise, it is not particularly limited to the above content.

Meanwhile, in another embodiment of the present inventive concept, the control unit 120 may include a CPU and a memory, which may be formed in one chip. The CPU may include a control circuit, an operation unit (ALU), an instruction interpretation unit, a registry, etc., and the memory may include RAM. The control circuit may perform a control operation, and the operation unit may perform an operational operation of binary bit information, and the instruction interpretation unit may include an interpreter, a compiler, etc. to convert high-level language into machine language or machine language into high-level language, and the registry may be involved in software-wise data storing. According to the above configuration, for example, the Al-based automatic 3D face scan matching device 100, at the operation initial stage, duplicates a program stored in the Al-based automatic 3D face scan matching unit 130 to load the program on a memory, that is, RAM, and execute the same, thereby rapidly increasing a data operation processing speed. For a case of a deep learning model, the program is loaded on a GPU memory, not RAM, thereby executing the program by accelerating a performance speed using the GPU.

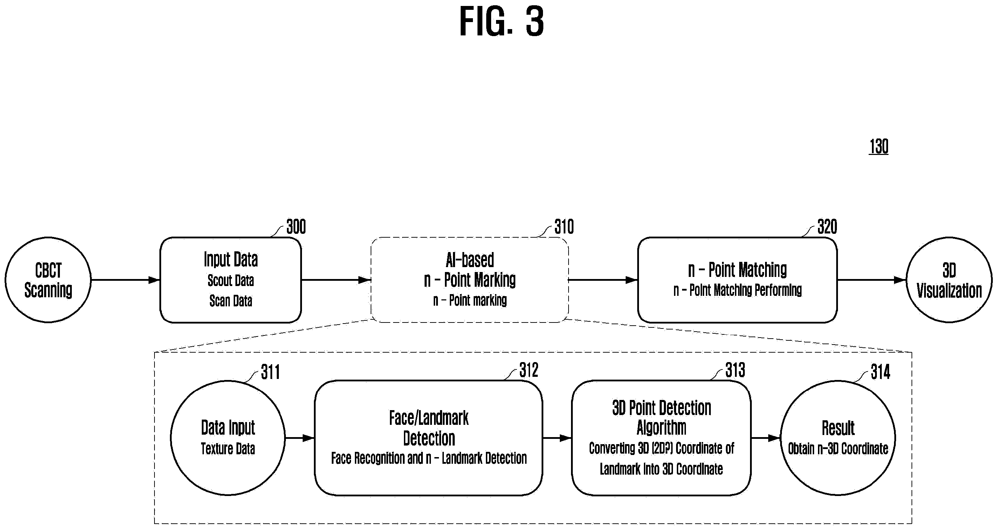

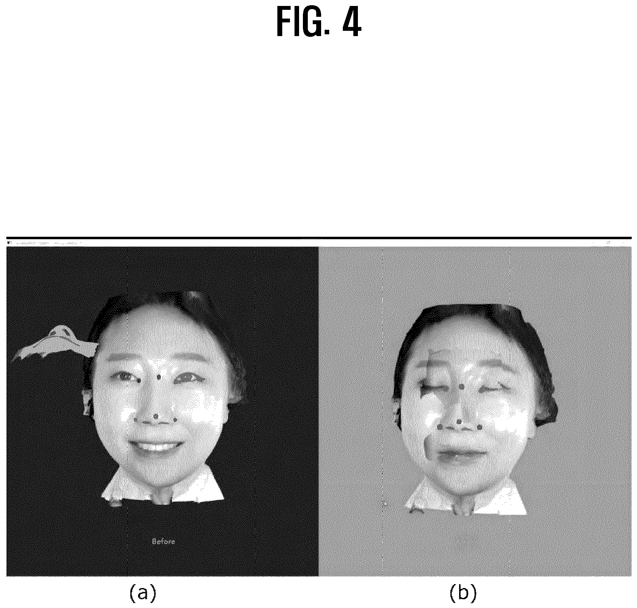

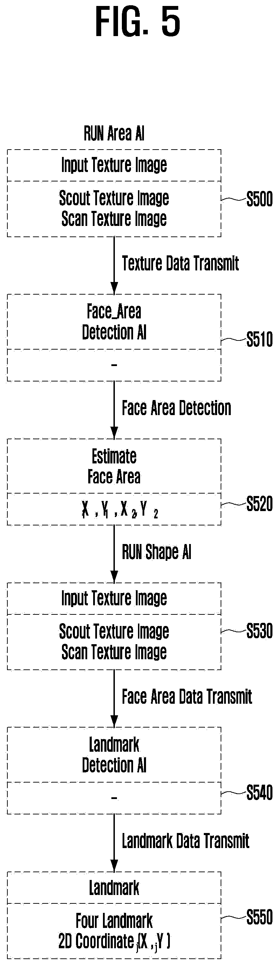

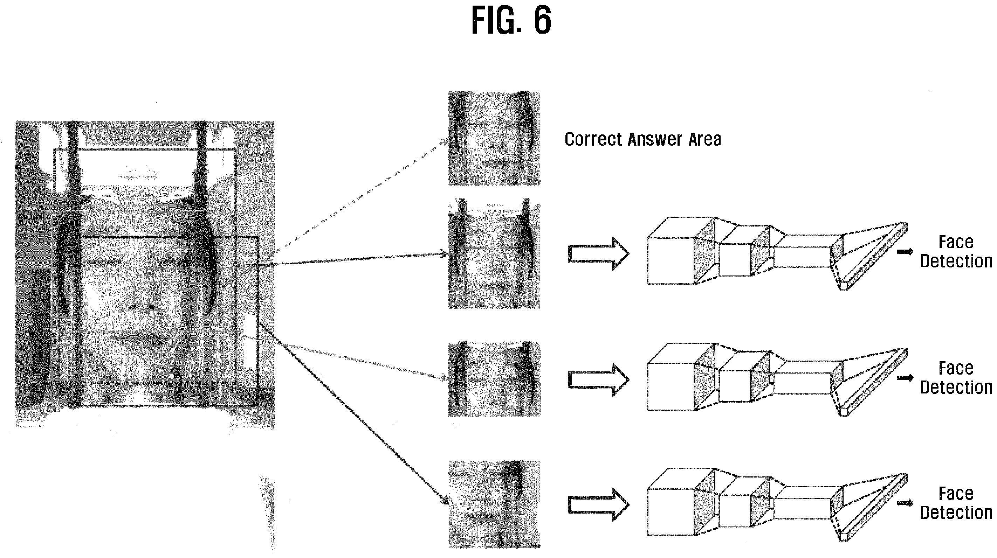

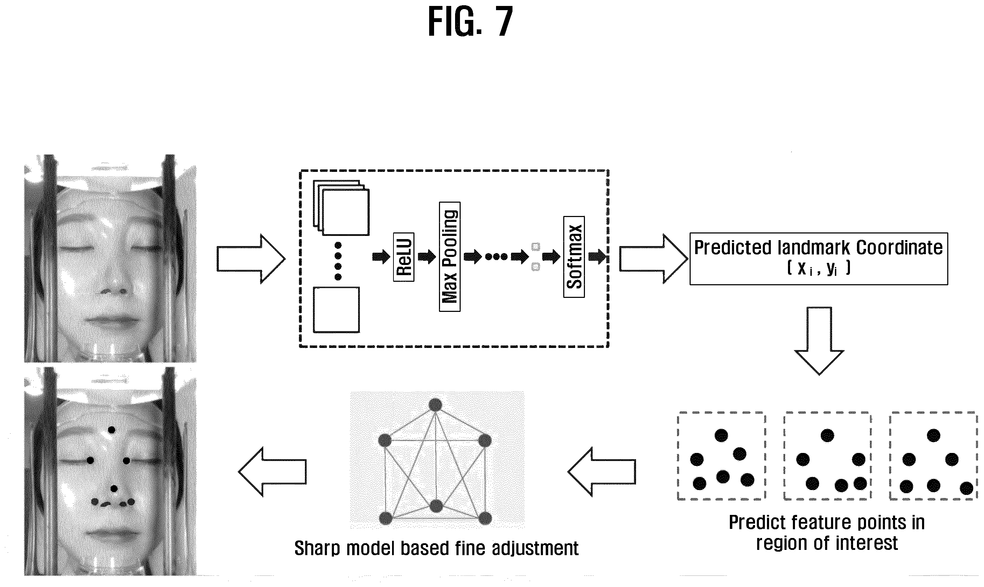

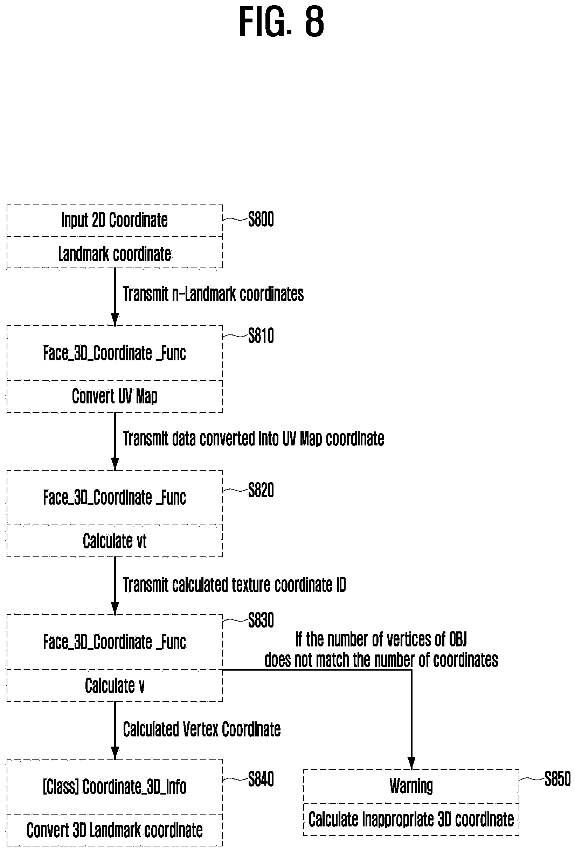

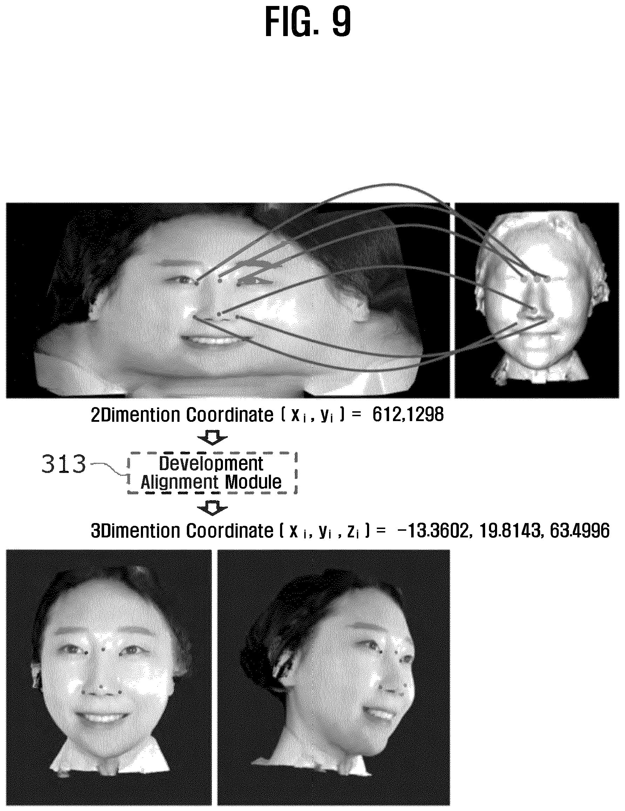

FIG. 3 is a block diagram showing an example of a detailed structure of Al-based automatic 3D face scan matching unit of FIG. 1. FIG. 4 is a view for explaining a comparison between a manual method and an automatic method having artificial intelligence applied thereto when performing marking (or detection) on a detection point. FIG. 5 is a view for explaining a detailed operation of a face and landmark detection unit of FIG. 3. FIG. 6 is a view for explaining operations S500 to S520 of FIG. 5. FIG. 7 is a view for explaining operation S540 of FIG. 5. FIG. 8 is a view for explaining a detailed operation of a 3D point detection unit of FIG. 3. FIG. 9 is a view for explaining operation S800 to operation S850 of FIG. 8.

As illustrated in FIG. 3, the Al-based automatic 3D face scan matching unit 130 of FIG. 1 according to an embodiment of the present inventive concept may include some or all of a data receiving unit 300, an Al-based detection point marking unit 310, a detection point matching unit 320, and a 3D visualization unit.

Here, the words "including some or all " means that the Al-based automatic 3D face scan matching unit 130 is configured by omitting some components, such as the data receiving unit 300 or the 3D visualization unit, or some components, such as the detection point matching unit 320 is integrated into the Al-based detection point marking unit 310, etc. To help sufficient understanding of the present inventive concept, in this description, it is assumed that all components are included.

The data receiving unit 300 may receive the SCOUT image data from a CBCT scanning device, and the face scan image data from a face scanning device. In addition, the data receiving unit 300 may further receive three-dimensional CT image data for 3D visualization, that is, visualization data generation. In an embodiment of the present inventive concept, by matching two-dimensional face scan image data to three-dimensional CT image data, a CT image for a smiling face of a patient may be seen so as to enable accurate diagnosis.

The Al-based detection point marking unit 310, as can be seen in FIG. 3, includes some or all of a data input unit 311, a face and landmark detection unit 312, a 3D detection point (or feature point) detection unit 313, and a result output unit 314. Here, the words "including some or all" are not much different from the meaning described above. The data input unit 311, the face and landmark detection unit 312, the 3D detection point detection unit 313, and the result output unit 314 may be configured with hardware, software, or a combination thereof, and in an embodiment of the present inventive concept, a configuration with SW may be preferable, but not particularly limited to any one form.

The face and landmark detection unit 312, as can be seen in FIGS. 4 to 7, may automate matching of the SCOUT data and the face scan data through artificial intelligence. In other words, approximately 27 detection points or feature points may exist in the human face. In this state, in an embodiment of the present inventive concept, a point that does not move even when blinking, that is, a point of which position is not changed in the face, is used as a landmark, and FIGS. 5 to 7 show a process thereof. In other words, the face and landmark detection unit 312 performs a face area detection operation to perform operation S520 of FIG. 5. FIG. 6 illustrates a feature point detection process for automatic matching in a texture image. The process may be used when a degree of overlapping of an input correct answer area, that is, an area predicted by the reference information and an artificial intelligence program (or algorithm) is 50% or more. Artificial intelligence proposes a face area by repeating the process as shown in FIG. 6. In other words, for face recognition, in an embodiment of the present inventive concept, with respect to the input SCOUT image data, feature points may be detected by setting a plurality of areas or changing the set areas, and by learning the process through artificial intelligence, the face area may be accurately recognized through the detected feature points.

Next, the face and landmark detection unit 312 may perform an operation of operation S540 of FIG. 5. In other words, when a face area is recognized, for example, a point that does not move even when smiling or blinking, that is, a landmark, is detected from the corresponding face area. FIG. 7 is a view for explaining landmark detection AI. In an embodiment of the present inventive concept, among 51 feature points to be detectable in the face, feature points that can be used as landmarks may be limited to 4 to 6 for use. As can be seen in FIG. 7, 2 inner corners of the eyes, the tip of the nostrils, and the exact center portion between the eyebrows may be used. Accordingly, landmark detection accuracy may be increased by determining or learning the shape regarding 6 points. Accordingly, the face and landmark detection unit 312 may output 6 landmark two-dimensional coordinates from the SCOUT image data and the face scan image data. In other words, it may be said that, as the SCOUT image is the same as a patient's actual face ratio, the face and landmark detection unit 312 detects a landmark from the face scan image data by using the same.

Furthermore, the 3D detection point detection unit 313 of FIG. 3 performs an operation of recalculating, that is, converting, the 2D coordinate of a landmark into a 3D coordinate. FIGS. 8 and 9 respectively illustrate detailed operation flow and shape performed in the 3D detection point detection unit 313 of FIG. 3. The 3D detection point detection unit 313 converts n, for example, 6 landmark coordinates, into 3D coordinates based on the data of converting the face scan image data into a UV map coordinate. For this purpose, the 3D detection point detection unit 313 may calculate a shape or a form made by V, that is, three-dimensional coordinates or check VT, that is, color information regarding the position in the texture image. More accurately, the face scan image data and the 3D face scan image data which are converted after the face scan image data is converted into the UV map coordinate include coordinate values, and thus, the two-dimensional coordinate of a landmark is converted into a three-dimensional coordinate. This process is well illustrated in FIG. 9.

Various operations may be further performed in the conversion process into a three-dimensional coordinate. For example, in a process of calculating V, when the number of vertices of OBJ does not match the number of texture coordinate values, it is determined to be an inappropriate 3D coordinate so as to be recognized as a warning. In this case, the corresponding coordinate value is disregarded and an operation for coordinate value calculation may be re-performed. The UV map coordinate may be a map coordinate that may be obtained after covering two-dimensional face scan image data into three-dimensional image data using a separate algorithm, etc. In other words, UV mapping is a three-dimensional modeling process of creating a two-dimensional image into a three-dimensional model, and for example, the simplest UV mapping may consist of three steps: a mesh decomposition step, a texture creation step, and a texture application step. Accordingly, for example, as can be seen in FIG. 9, the 3D face scan data may be overlapped with the UV map data obtained by converting the two-dimensional face scan image data, and thus, it is possible to extract a three-dimensional coordinate value for the landmark two-dimensional coordinate value.

As described above, the 3D detection point detection unit 313 converts n landmark coordinates in three-dimensional coordinates through the UV map and calculates V and VT for the converted three-dimensional coordinates, and when it is determined that there is no problem in the calculation, for example, the number of vertices of OBJ is the same as the number of texture coordinate values, converts the three-dimensional coordinates into 3D landmark coordinates. For example, when the number of vertices of OBJ does not match the number of texture coordinate values, three-dimensional coordinates is determined into inappropriate 3D coordinates so that a separate conversion operation may not be performed. FIG. 9 illustrates a process in which coordinate conversion is performed through the 3D detection point detection unit 313.

The detection point matching unit 320 matches (or overlaps) the face scan image data to the three-dimensional CT image based on the detection points of landmarks converted into the three-dimensional coordinate values so as to see a CT image for a smiling face of a patient. Accordingly, by matching the face scan image data to the three-dimensional CT image, when a patient smiles, a bone structure, a degree of exposure of teeth, etc. is changed so that accurate diagnosis may be achieved.

As a result, although the SCOUT image has an actual ratio of the face of a patient, in the dental or cosmetic treatment, it may be more interested in accurately recognizing a gum shape for a smiling face of a patient, and in how a prosthesis and a denture affects In this state. To this end, based on the SCOUT image data, landmarks, that is, detection points that do not move even when the eyes blink, are found in the 3D face scan image data, and by matching the face scan image to the CT scanning image based on those detection points, a CT image for a smiling face can be visualized in 3D. In this process, as coordinate values for the detection points of landmarks detected from the face scan image data are two-dimensional, the corresponding coordinates are converted into three-dimensions and matched to the CT image through the converted three-dimensional coordinate values, thereby enabling 3D visualization.

In this process, in an embodiment of the present inventive concept, artificial intelligence is applied to recognizing a face and detecting the detection points of landmarks through feature points detected from the face. Through the artificial intelligence, the landmark detection points are detected at accurate positions in the face. Accordingly, the detection or marking of a consistent position is performed so that accuracy of matching may be increased. In addition, in a case of matching or detection, the face scan image is converted into the UV map, and thus, based on this, the three-dimensional coordinate values for the two-dimensional landmark detection points are obtained. In the process of determining whether to convert into the three-dimensional coordinate value, by determining a shape formed with a coordinate value or checking color information, the conversion of three-dimensional coordinate values for the coordinate values of two-dimensional landmark detection points is finally determined based on the checking or determining result.

When the conversion into the three-dimensional coordinates is made through this process, by matching the face scan data to the CT scanning image data, 3D visualization of the CT image through a smiling face is achieved. As accurate coordinate values may be detected only when accurate marking or detection is made for the detection points of landmarks, the corresponding detection operation is performed by analyzing pixel information, etc. as the feature of region of interest, for example, reference information, through artificial intelligence or learning. Accordingly, marking or detection for the detection points of landmarks at accurate positions is possible. Through artificial intelligence, accurate detection corresponding thereto is made based on a reference point or a reference value. For example, as the inner corners of the eyes, as reference points, may have designated pixel characteristics, information thereon may be preset, and artificial intelligence may detect an accurate position by comparing a result of the pixel analysis with the preset information.

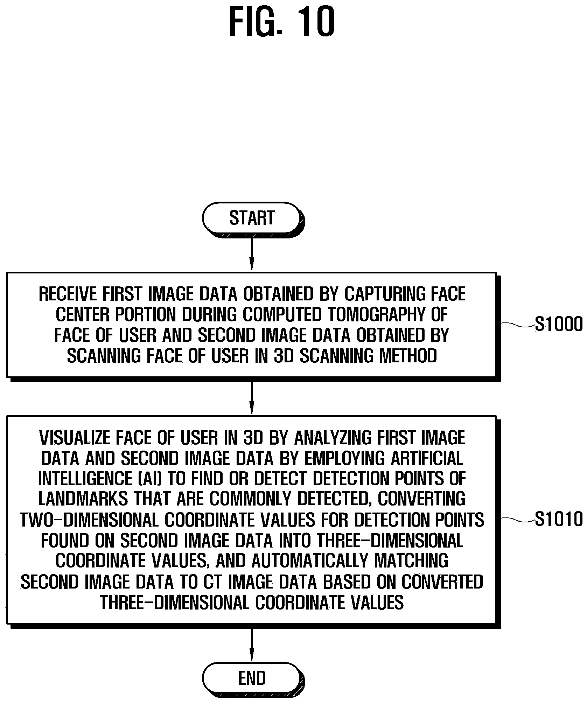

FIG. 10 is a flowchart showing a driving process of the Al-based automatic 3D face scan matching device of FIG. 1.

For convenience of explanation, referring to FIG. 10 with FIG. 1, the Al-based automatic 3D face scan matching device 100 of FIG. 1 according to an embodiment of the present inventive concept receives the first image data obtained by capturing a face center portion during the computed tomography of the face of a user, and the second image data obtained by scanning the face of a user in a 3D scanning method (S1000). Here, it may be seen that the first image data is the SCOUT image data that is a captured image of the face center portion during CT scanning as in (a) of FIG. 2, and the second image data is the image data of a face scan image as in (b) of FIG. 2. In this process, Three-dimensional image data for the CT image and three-dimensional image data for the face scan may be received or generated.

Furthermore, the Al-based automatic 3D face scan matching device 100 visualizes the face of a user in 3D by analyzing the first image data and the second image data by having artificial intelligence (AI) applied thereto to find or detect detection points of landmarks that are commonly detected, converting two-dimensional coordinate values for the detection points found on the second image data into three-dimensional coordinate values, and automatically matching the second image data to the CT scanning image data based on the converted three-dimensional coordinate values (S1010).

To be more exact, the Al-based automatic 3D face scan matching device 100 may perform accurate detection on the detection points of the SCOUT image data, that is, the detection points of landmarks, and the detection points of landmarks in the face scan image data, by executing an artificial intelligence program, based on the preset reference point or the reference information. The Al-based automatic 3D face scan matching device 100 may convert the coordinate values extracted based on the detection points of landmarks into three-dimensional coordinates by using the face scan image data (e.g.: converting into the UV map data) or using the three-dimensional data of the face scan image, and match the face scan image data to the three-dimensional CT scanning data based on the converted three-dimensional coordinates, thereby enabling seeing a CT image for a smiling face of a patient. This may be possible through 3D visualization.

In addition to the content described above, the Al-based automatic 3D face scan matching device 100 of FIG. 1 may perform various operations, and as other detailed contents are sufficiently described above, redundant descriptions are omitted.

Although embodiments have been described above in detail, the scope of the present disclosure is not limited thereto, and various modifications and alterations by those skill in the art using the basic concept of the present disclosure defined in the following claims also fall within the scope of the present disclosure, and such modified embodiments should not be individually interpreted from the technical concept or prospect of the present inventive concept.

Meanwhile, although it has been described in the above that all the components of an embodiment of the present inventive concept are coupled as a single unit or coupled to be operated as a single unit, the disclosure is not necessarily limited to such an embodiment. Namely, within the purpose of the disclosure, one or more components among the components may be selectively coupled to be operated as one or more units. Also, although each of the components may be implemented as an independent hardware, some or all of the components may be selectively combined with each other, so that they may be implemented as a computer program having one or more program modules for performing some or all of the functions combined in one or more hardware. Codes and code segments forming the computer program can be easily conceived by an ordinarily skilled person in the technical field of the disclosure. Such a computer program may implement the embodiments of the disclosure by being stored in a non-transitory computer-readable medium, and being read and executed by the computer.

Here, the non-transitory readable medium does not refer to a medium, for example, register, cache, memory, etc. which stores data for short time, but refers to a medium that semi-permanently stores data and readable by a device. In detail, the programs described above may be stored and provided on a non-transitory readable recording medium, such as CD, DVD, hard disks, Blu-ray discs, USB, memory cards, ROM, etc.

While this inventive concept has been particularly shown and described with reference to preferred embodiments thereof, it will be understood by those of ordinary skill in the art that various changes in form and details may be made therein without departing from the spirit and scope of the inventive concept as defined by the appended claims. Therefore, the scope of the inventive concept is defined not by the detailed description of the inventive concept but by the appended claims, and all differences within the scope will be construed as being included in the present inventive concept.

### INDUSTRIAL APPLICABILITY

The present inventive concept may be used for a medical industry, in particular, a dental medical industry.

## Claims

1. An automatic three-dimensional face scan matching device having artificial intelligence applied thereto, the automatic three-dimensional face scan matching device comprising: a communication interface unit receiving first image data obtained by capturing a face center portion during computed tomography (CT) scanning of a face of a user and second image data obtained by scanning the face of a user in a 3D scanning method; and a control unit visualizing the face of a user into 3D by analyzing the first image data and the second image data by having artificial intelligence (AI) applied thereto to find detection points of landmarks that are commonly detected, converting two-dimensional coordinate values for the detection points found on the second image data into three-dimensional coordinate values, and automatically matching the second image data to CT scanning image data based on the converted three-dimensional coordinate values.

2. The device of claim 1, wherein the control unit analyzes the first image data and the second image data by having the artificial intelligence applied thereto to extract a plurality of detection points related to feature points respectively therefrom, and extracts a plurality of landmark detection points that are common with each other from among the extracted plurality of detection points.

3. The device of claim 2, wherein the control unit extracts, as the landmark detection points, detection points where the feature points are not changed in a face portion during the CT scanning and a face portion during the 3D scanning.

4. The device of claim 2, wherein the control unit presets reference information to detect the feature points and the plurality of landmark detection points, and detects the feature points and the landmark detection points by applying the artificial intelligence thereto based on the preset reference information.

5. The device of claim 4, wherein the control unit determines a shape formed with the feature points as the reference information, and finalizes the landmark detection points based on a result of the determination.

6. The device of claim 1, wherein the control unit converts the second image data into three-dimensional UV map data, converts two-dimensional coordinate values for the detection points into the three-dimensional coordinate values by using the converted UV map data and three-dimensional data generated during the face scanning, by determining a shape formed with three coordinates during the conversion of the three-dimensional coordinate values and color information of each coordinate, and performs conversion of the three-dimensional coordinate value based on a result of the determination.

7. A method for driving an automatic three-dimensional face scan matching device having artificial intelligence applied thereto, the method comprising: receiving, performed by a communication interface unit, first image data obtained by capturing a face center portion during computed tomography scanning of a face of a user and second image data obtained by scanning the face of a user in a 3D scanning method; and visualizing, performed by a control unit, the face of a user into 3D by analyzing the first image data and the second image data by having artificial intelligence applied thereto to find detection points of landmarks that are commonly detected, converting two-dimensional coordinate values for the detection points found on the second image data into three-dimensional coordinate values, and automatically matching the second image data to CT scanning image data based on the converted three-dimensional coordinate values.

8. The method of claim 7, wherein the visualizing comprises: extracting a plurality of detection points related to feature points respectively therefrom by analyzing the first image data and the second image data by having the artificial intelligence applied thereto; and extracting a plurality of landmark detection points that are common with each other from among the extracted plurality of detection points.

9. The method of claim 8, wherein the extracting of the plurality of landmark detection points comprises extracting, as the landmark detection points, detection points where the feature points are not changed in a face portion during the CT scanning and a face portion during the 3D scanning.

10. The method of claim 8, wherein the visualizing comprises: presetting reference information for detecting the feature points and the plurality of landmark detection points; and detecting the feature points and the landmark detection points by applying the artificial intelligence thereto based on the preset reference information.

11. The method of claim 10, wherein the detecting of the landmark detection point comprises determining a shape formed with the feature points as the reference information, and finalizing the landmark detection points based on a result of the determination.

12. The method of claim 7, wherein the visualizing comprises: converting the second image data into three-dimensional UV map data; converting two-dimensional coordinate values for the detection points into the three-dimensional coordinate value by using the converted UV map data and three-dimensional data generated during the face scanning; and determining a shape formed with three coordinate values during the conversion of the three-dimensional coordinate values and color information of each coordinate, and performing conversion of the three-dimensional coordinate values based on a result of the determination.

13. A computer program stored in a medium to perform an automatic three-dimensional face scan matching method by having artificial intelligence applied thereto, wherein the automatic three-dimensional face scan matching method by having artificial intelligence applied thereto comprises: receiving first image data obtained by capturing a face center portion during computed tomography scanning of a face of a user and second image data obtained by scanning the face of a user in a 3D scanning method; and visualizing the face of a user into 3D by analyzing the first image data and the second image data by having artificial intelligence applied thereto to find detection points of landmarks that are commonly detected, converting two-dimensional coordinate values for the detection points found on the second image data into three-dimensional coordinate values, and automatically matching the second image data to CT scanning image data based on the converted three-dimensional coordinate values.
